# EUROPEAN PATENT APPLICATION

(11) **EP 1 491 624 A1**
(43) Date of publication of application: **29.12.2004**
(21) Application number: 02714467.4
(22) Date of filing: 04.04.2002
(51) Int. Cl.: C12N 5/08

(54) **METHOD OF CULTURING LIVER CELLS OVER LONG TIME**

(71) Applicant: DAIICHI PURE CHEMICALS CO., LTD., Chuo-ku Tokyo 103-0027 (JP)
(72) Inventor: SHIMADA, Noriaki Daiichi Pure Chemicals co. ltd, Ibaraki 319-1112 (JP); MAUREL, Patrick, F-34293 Montpellier Cedex 05 (FR)
(74) Representative: Hartz, Nikolai F., Dr.
(86) International application number: PCT/JP2002/003398
(87) International publication number: WO 2003/085100

(57) **Abstract**

A method of culturing hepatocytes for a long term, characterized in that fresh hepatocytes are maintained in a medium at 15 to 30°C for 1 to 6 days, followed by culturing under physiological conditions.

The method of the present invention enables hepatocytes to be cultured for a long term without inviting reduction in enzymatic activity or enzyme-inducing activity. Moreover, since the cells can be maintained at around room temperature for 1 to 6 days, the method is very useful for long-term transportation of hepatocytes after isolation.

## Description

### Technical Field

The present invention relates to a method of culturing fresh hepatocytes over a long period of time while maintaining enzymatic activities and enzyme-inducing activities of the cells.

### Background Art

Hepatocytes produce not only albumin, but also drug metabolizing enzymes such as those belonging to the cytochrome P450 (CYP) family, and coagulation-fibrinolysis-related enzymes such as α1-antitrypsin. Therefore, culture products of fresh hepatocytes are widely used for measuring the activities of such enzymes.

Typically, hepatocytes that are collected from the liver are stored in a cool place at 4°C or lower so as not to permit deactivation of enzymes, and thereafter, the temperature is returned to about 37°C and the cells are cultured for a long period of time before they are used for performing a variety of tests.

In recent years, as human hepatocytes have become difficult to obtain, a need arises for acquired hepatocytes to be stored for a long period of time or transported a long distance (e.g., cross-border transportation). To meet such need, fresh hepatocytes must be stored over at least 2 days without inviting any loss of enzymatic activities or enzyme-inducing activities.

However, in conventional low-temperature storage at not higher than 4°C, enzyme activities and enzyme-inducing activities are generally lowered by 30 to 60% during storage of 2 or more days, thus hampering correct measurement of such activities.

Accordingly, an object of the present invention is to provide a method of culturing hepatocytes over a long period of time without permitting reduction in enzymatic activities or enzyme-inducing activities of the cells.

### Disclosure of the Invention

The present inventors have studied conditions under which fresh hepatocytes can be satisfactorily cultured for a long term, and have obtained the following findings. That is, when hepatocytes are stored at about at 37°C, which represents a physiological condition, enzymatic activities and enzyme-inducing activities of the cells are maintained at certain appreciable levels as compared with the case where the cells are stored at a conventionally employed low temperature (4°C). However, when the cells are stored at room temperature; specifically between 15 and 30°C, for 1 to 6 days, and then returned to physiological conditions and cultured, quite unexpectedly, even higher enzymatic activities and enzyme-inducing activities are retained as compared with the case of the. storage at 37°C, and the activities remain persistent for long periods of time, and when the hepatocytes are transported a long distance, biological tests such as drug metabolism enzyme-inducing tests are performed accurately. The present invention has been accomplished on the basis of these findings.

Accordingly, the present invention provides a method of culturing hepatocytes for a long term, characterized in that fresh hepatocytes are maintained in a medium at 15 to 30°C for 1 to 6 days, followed by culturing under physiological conditions.

### Brief Description of the Drawings

Fig. 1 shows the urea synthesis (i.e., ureagenesis) capacity of monkey hepatocytes which were cultured after storage for 48 hours under a variety of conditions.
Fig. 2 shows the α1-antitrypsin synthesis capacity of monkey hepatocytes which were cultured after storage at 25°C for 48 hours.
Fig. 3 shows the α1-antitrypsin synthesis capacity of monkey hepatocytes which were cultured after storage at 25°C for 96 hours.
Fig. 4 shows the CYP3A4 inducing capacity of monkey hepatocytes which were cultured after storage at 25°C for 48 hours (RIF represents exposure to rifampicin, whereas UT represents non-exposure to rifampicin).
Fig. 5 shows the urea synthesis (i.e., ureagenesis) capacity of human hepatocytes which were cultured after storage at 25°C for 48 hours.
Fig. 6 shows the urea synthesis capacity of human hepatocytes which were cultured after storage at 25°C for 96 hours.
Fig. 7 shows the urea synthesis capacity of human hepatocytes which were cultured after storage at 25°C for 144 hours.
Fig. 8 shows the albumin synthesis capacity of human hepatocytes which were cultured after storage at 25°C for 96 hours.
Fig. 9 shows the α1-antitrypsin synthesis capacity of human hepatocytes which were cultured after storage at 25°C for 96 hours.
Fig. 10 shows the CYP3A4 inducing capacity of human hepatocytes which were cultured after storage at 25°C for 48 hours, 96 hours, or 144 hours (RIF represents exposure to rifampicin, whereas UT represents non-exposure to rifampicin).

### Best Modes for Carrying Out the Invention

The hepatocytes to which the method of the present invention is applied are fresh hepatocytes which have not been subjected to subculturing after removal from the organism. In other words, they are hepatocytes of primary culture. Examples of the hepatocytes include those from a mammal, in particular, those from the primates. Human hepatocytes are very much preferred.

According to the present invention, fresh hepatocytes must be maintained in a medium at 15 to 30°C for 1 to 6 days. When the hepatocytes are maintained at a temperature below 15°C, for example, at 4°C (which is the conventional storage temperature), the enzymatic activities and enzyme-inducing activities of the hepatocytes decrease. Likewise, when the hepatocytes are maintained at a temperature higher than 30°C, for example, at about 37°C (which is a physiological condition), the enzymatic activities and enzyme-inducing activities of the hepatocytes decrease even further as compared with the case where the cells are stored at 15 to 30°C. The temperature at which the hepatocytes are maintained is preferably 18 to 27°C, more preferably 20 to 25°C.

The medium to be used in the present invention for maintaining fresh hepatocytes may be a conventional one used for animal cells. Examples of such a medium include Lanford's medium, William's E (WME) medium, Isom's medium, Leibovitz L15 medium, polyethylene-glycol-added Leibovitz L15 medium, and any combinations thereof. A particularly preferred medium contains at least Lanford's medium.

The number of the hepatocytes added to a medium for maintaining fresh hepatocytes is 0.5×10⁶ to 10×10⁶ cell/mL, preferably 1×10⁶ to 10×10⁶ cell/mL.

The period of time during which the hepatocytes are maintained at 15 to 30°C is 1 to 6 days, preferably 2 to 6 days.

After the hepatocytes are maintained at 15 to 30°C for 1 to 6 days, the cells are cultured under physiological conditions. Preferably, such a culture under physiological conditions is performed in a conventional medium at 37±1°C. As used herein, no particular limitation is imposed on the medium so long as it can be used for culturing conventionally employed animal cells. Preferably, the medium is the same as that employed for maintaining the aforementioned fresh hepatocytes. In particular, use of a medium containing at least the Lanford's medium is preferred.

When hepatocytes are subjected to continuous culturing under physiological conditions as described above, the enzymatic activities and enzyme-inducing activities of the cells do not drop over a prolonged period; i.e., over one month or more, and therefore, the hepatocytes can be used for accurate measurement of enzymatic activities or drug metabolizing enzyme-inducing activities. Examples of measurable enzymatic activities include α1-antitrypsin activity, glutamine-S-transferase (GST), GOT, GPT, ureagenesis, and albumin synthesis capacity. Example of enzyme-inducing activities includes those for inducing enzymes belonging to the CYP family, such as CYP1A1, CYP1A2, CYP2A6, CYP2B6, CYP2C8, CYP2C9, CYP2C19, CYP2E6, and CYP3A4.

Procurement of hepatocytes, in particular human hepatocytes, is not easy. Particularly when the site where hepatocytes are obtained from an organism is remote from the sites where tests are to be carried out, as in the case of cross-border situation, transportation of hepatocytes would typically need 2 or more days. The method of the present invention is very suitable in cases where tests are performed after long-distance transportation of the cells.

### Examples

The present invention will next be described in more detail by way of examples, which should not be construed as limiting the invention thereto.

### A. Materials and Methods

### (1) Primary culture of hepatocytes

Human livers were removed from four patients. Monkey livers were obtained from young male monkeys.

Human hepatocytes were collected from the liver that had been subjected to lobectomy through a known method (Drug Metab. Dispos. 18: 595-606 (1990), Mol. Pharmacol. 41: 1047-1055 (1992), J. Pharmacol. Exp. Ther. 269: 384-392 (1994)), and cultured. Viability of the cells to be plated was checked by the trypan blue exclusion test, and was found to be 80 to 90%. The hepatocytes were seeded in Isom's medium placed in a culture flask (25 cm²) coated with collagen I (5 µg/cm²) so as to attain confluency (12.4×10⁴ cells/cm²). The medium employed was a 1:1 mixture of Ham F12 and William's E, which was prepared as described in Proc. Nat. Acad. Sci. USA; 81: 6378-6382 (1984). During the first 4 hours after the cells were seeded, 5% calf serum was added to thereby promote adhesion of cells. Four hours after plating of hepatocytes, the calf-serum-added standard medium was removed, and instead, Lanford's medium was added. Thus, on day 1 of culture, the medium was exchanged, and subsequently, the culture product of hepatocytes were maintained under humid conditions of 5% CO₂ at 37°C for 3 or 4 days. In this manner, once a hepatocyte culture system was established, the Lanford's medium was removed through aspiration. The interior of the flask was purged with 5%-CO₂-equilibrated 95% air, and filled with the Lanford's medium (abbreviated as "Lnf"), 5% polyethylene glycol-added Leibovitz L15 (Sigma Chemical Co.) medium (abbreviated as "L15 + 5% PEG"), or Isom's medium (37°C). The flask was then maintained for 2, 4 or 6 days under ice-cold conditions or at room temperature. Subsequently, the medium of the hepatocyte-containing flask was again changed to Lnf at 37°C for long-term culturing. In order to assess the effects of medium, temperature, and storage period on the viability and functions of hepatocytes, the cells collected 24 hours after every medium exchange were tested for several parameters representing the phenotypes of the cells. Specifically, the tested parameters included urea synthesis capacity (through enzymatic/spectrometric analysis), and α1-antitrypsin- or albumin-synthesizing capacity (through western blotting). The results are shown in a comparative manner with the initial value and the data of the control group (control: a group which was maintained at 37°C in Lnf during the test period). Moreover, response of cells in terms of induction of cytochrome P450 enzyme (induction of CYP3A4 by rifampicin) was also studied. This response is considered an index which best tells whether or not liver-specific phenotypes are maintained, and therefore, serves as a critical factor in development of drugs, as it can predict drug interaction.

### (2) Medium composition

The medium compositions employed are shown in Tables 1, 2, and 3.

**Table 1**

| Lanford's medium (Lnf) | |
|---|---|
| 0.5 mg/mL | BSA |
| 1 µM | Hydrocortisone |
| 10 µg/mL | Insulin |
| 50 ng/mL | Epidermal growth factor (EGF) |
| 2 µg/mL | Glucagon |
| 5 µg/mL | Linolenic acid |
| 0.1 µM | Selenium acetate |
| 2 ng/mL | Cholera toxin |
| 20 ng/mL | Hepatocyte growth factor |
| 5 µg/mL | Transferrin |
| 1 µM | Ethanolamine |
| 100 ng/mL | Prolactin |
| 100 U/mL | Penicillin |
| 100 µg/mL | Streptomycin |
| 25 µg/mL | Fungizone |

**Table 2**

| | |
|---|---|
| L15 + 5% PEG | |

| Leibovitz L15 (product of Sigma Chemical Co.) 5% Polyethylene glycol | |
|---|---|
| Leibovitz L15 | (mg/L) |
| Inorganic salt | |
| CaCl₂ | 140 |
| KCl | 400 |
| KH₂PO₄ | 60 |
| MgCl₂ | 94 |
| MgSO₄ | 8000 |
| NaCl | 190 |
| Na₂HPO₄ | |

| Other ingredients | |
|---|---|
| D(+)Galactose | 900 |
| Phenol red | 10 |
| Sodium pyruvate | 550 |

| Amino acid | |
|---|---|
| L-Alanine | 225 |
| L-Arginine | 500 |
| L-Asparagine | 250 |
| L-Cysteine | 120 |
| L-Glutamine | 300 |
| Glycine | 200 |
| L-Histidine | 250 |
| L-Isoleucine | 250 |
| L-Leucine | 125 |
| L-Lysine | 75 |
| L-Methionine | 75 |
| L-Phenylalanine | 125 |
| L-Serine | 200 |
| L-Threonine | 300 |
| L-Tryptophan | 20 |
| L-Tyrosine | 300 |
| L-Valine | 100 |

| Vitamins | |
|---|---|
| Calcium D-pantothenate | 1 |
| Choline chloride | 1 |
| Folic acid | 1 |
| Inositol | 2 |
| Niacinamide | 1 |
| Pyridoxine HCl | 1 |
| Riboflavin-5-phosphate 2H₂O | 0.1 |
| Thiamine monophosphate | 1 |

**Table 3**

| Isom's medium | |
|---|---|
| 2.4 mM | Glutamine |
| 0.1 µM | Dexamethasone (DEX) |
| 2 µg/mL | Insulin |
| 50 µg/mL | Transferrin |
| 3.6 µM | Linolenic acid |
| 66.8 µM | Ethanolamine |
| 0.4 mM | Sodium pyruvate |
| 250 µM | Ascorbic acid |
| 100 U/mL | Penicillin |
| 100 µg/mL | Streptomycin |
| 25 µg/mL | Fungizone |
| 0.5 µM | Fluconazole |

### (3) Preparation of lysate

From the cultured cells, lysate was prepared through a known method by use of a centrifuge, and stored until use. The protein concentration of the lysate was measured through the bicinchoninate method (Pierce Chemical Company).

### (4) Quantification of serum protein contained in an extracellular culture medium with which the hepatocytes are cultured

Quantification of albumin and α1-antitrypsin contained in an extracellular culture medium (5 to 20 µL medium) was performed through western blotting employing a human-protein-specific antibody. Medium samples were collected from 2 flasks (in which the cells were cultured). The collected samples were combined, followed by centrifuging at 11,000×g for 5 minutes and storing at -80°C until use for analysis.

### (5) Quantification of CYPs contained in cultured hepatocytes

In accordance with the method described in Hepatology 22: 1143-1153 (1995), CYPs were quantified by use of a specific polyclonal or monoclonal antibody through immunoblotting of a lysate. As a standard to be applied to each gel, a microsome containing genetically expressed CYP2C9, CYP2C19, or CYP3A4 (Gentest) was employed.

The lysate was subjected to electrophoresis (SDS 10% polyacrylamide gel) before transferring onto nitrocellulose membrane. The blots were allowed to develop by use of an ECL (Enhanced ChemiLuminescence) (Amersham). The relative amount of CYP was calculated through scanning by means of an LE program, and quantitative analysis by means of an NIH image 1.6/ppc program.

### (6) Urea synthesis capacity of the cultured hepatocytes contained in an extracellular culture medium

The urea synthesis capacity was measured by an enzymatic/spectrometric assay kit (Sigma Chemical Co.).

### B. Results

### (1) Effects of storage temperature on urea synthesis capacity

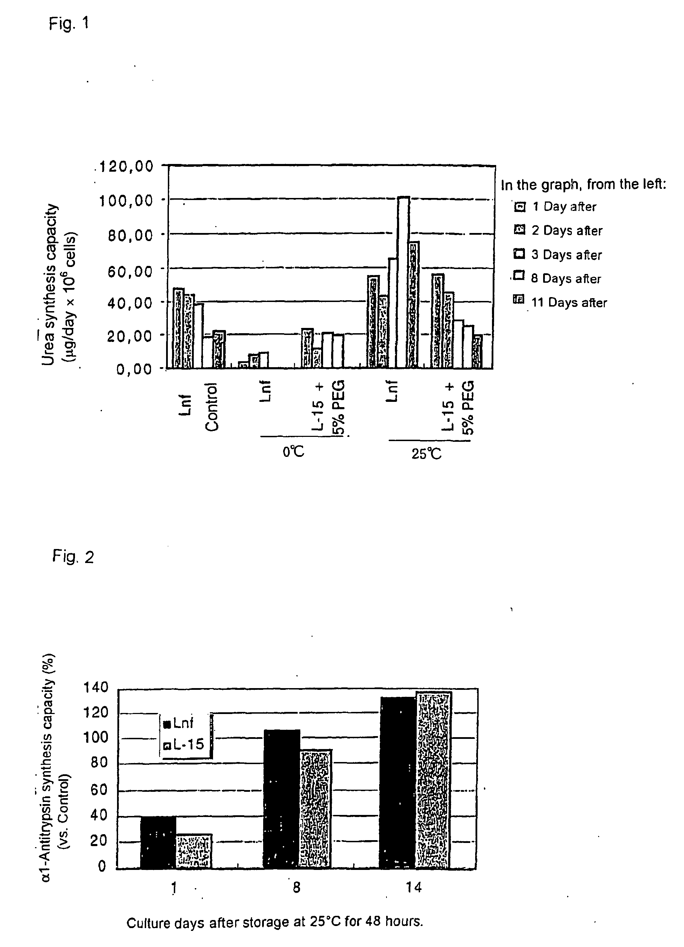

Monkey hepatocytes were maintained in Lnf or L-15 + 5% PEG for 2 days at 0°C or 25°C. Subsequently, the cells were cultured at 37°C in Lnf. The urea synthesis capacities of the monkey hepatocytes after culturing for 1, 2, 3, 8, or 11 days are shown in Fig. 1. As is clear from Fig. 1, as compared with the urea synthesis capacity of the cells of the control group which were cultured after having been stored at 37°C in Lnf, the urea synthesis capacities of the cell groups which were cultured after having been stored at 0°C were found to be lowered in both of the following cases; i.e., the case where the cells were cultured in Lnf and the case where the cells were cultured in L-15 + 5% PEG. In contrast, the cell groups which were cultured in Lnf following storage at 25°C and the cell groups which were cultured in L-15 + 5% PEG following storage at 25°C were both found to maintain urea synthesis capacities higher than those of corresponding control groups. Lnf provided more excellent results than L-15 + 5% PEG.

### (2) α1-Antitrypsin synthesis capacity and CYP3A4 inducing capacity

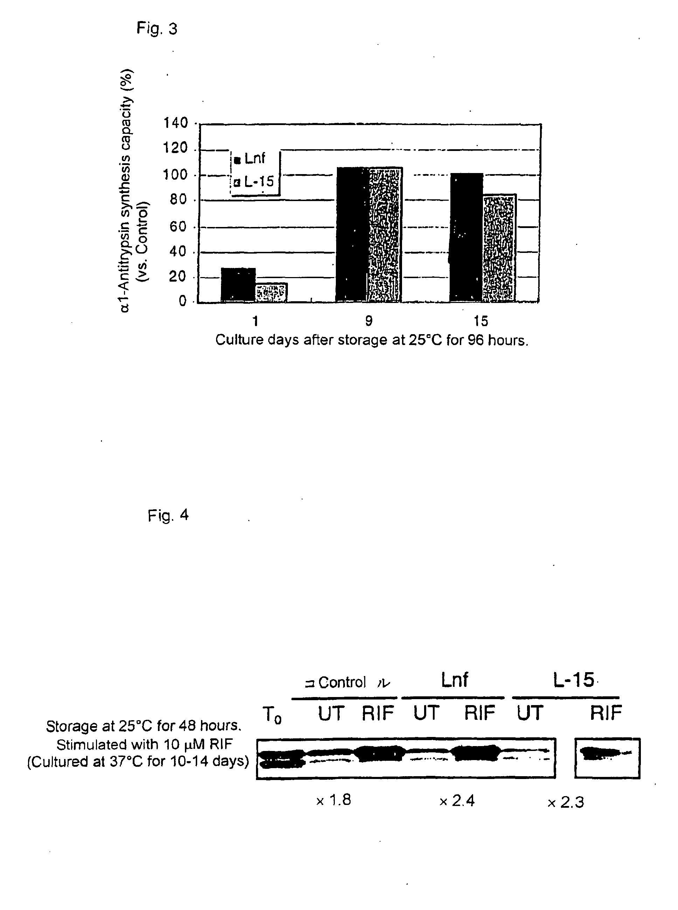

Monkey hepatocytes were maintained at 25°C in Lnf or in L-15 + 5% PEG for 2 days (48 hours) or 4 days (96 hours). subsequently, the cells were cultured in Lnf at 37°C, and α1-antitrypsin synthesis capacity of the cells was measured. The results are shown in Figs. 2 and 3. The data shown in Figs. 2 and 3 are relative values with respect to those obtained from the test where the cells were stored and cultured at 37°C from the beginning.

As is apparent from Figs. 2 and 3, in the case where cells were cultured after having been stored at 25°C for 2 or 4 days, as compared with the case where cells were stored and cultured at 37°C from the beginning, higher α1-antitrypsin synthesis capacity was maintained through the culture period of 8 to 15 days.

Separately, monkey hepatocytes were maintained in Lnf or L-15 + 5% PEG at 25°C for 2 days (48 hours). Subsequently, the cells were cultured in Lnf at 37°C, and the CYP3A4 inducing capacity of the cells was measured. The results are shown in Fig. 4. Fig. 4 also shows relative values obtained from the case where the cells were stored and cultured at 37°C from the beginning.

As is apparent from Fig. 4, in the case where the cells were cultured after having been maintained at 25°C for 2 days, strong CYP3A4 inducing capacities (2.3 times and 2.4 times) were observed on day 10 to day 14 of culture, as compared with the case where the cells were stored and cultured at 37°C from the beginning (1.8 times).

### (3) Effect on urea synthesis capacity (human hepatocytes)

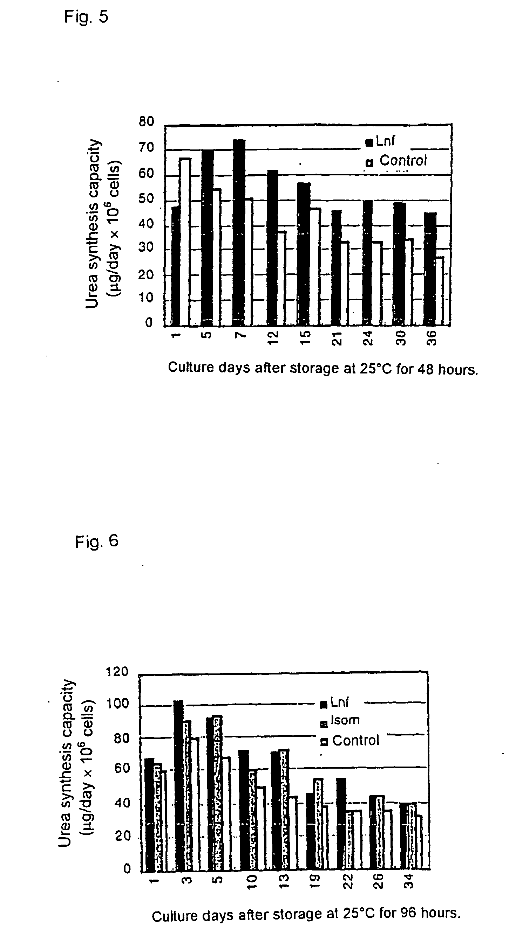

Human hepatocytes were maintained in Lnf or Isom at 25°C for 2, 4 or 6 days. Subsequently, the cells were cultured at 37°C in Lnf, and the urea synthesis capacity of the cells was measured. The results are shown in Figs. 5, 6, and 7.

As is apparent from Figs. 5, 6 and 7, as compared with the control groups in which the cells were maintained and cultured at 37°C from the beginning, the cells that were cultured after having been maintained in Lnf or Isom at 25°C for 2, 4 or 6 days exhibited strong urea synthesis capacities.

### (4) Albumin synthesis capacity and α1-antitrypsin synthesis capacity (human hepatocytes)

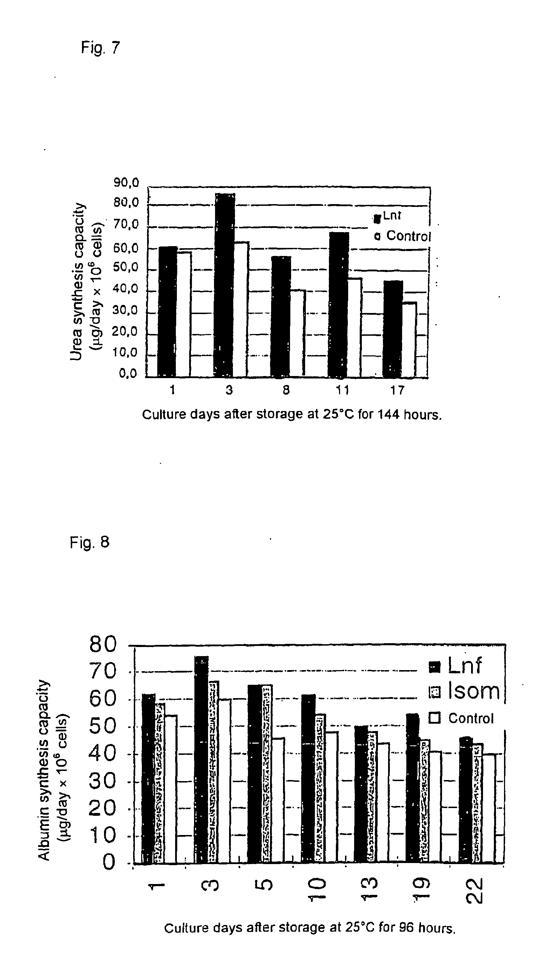

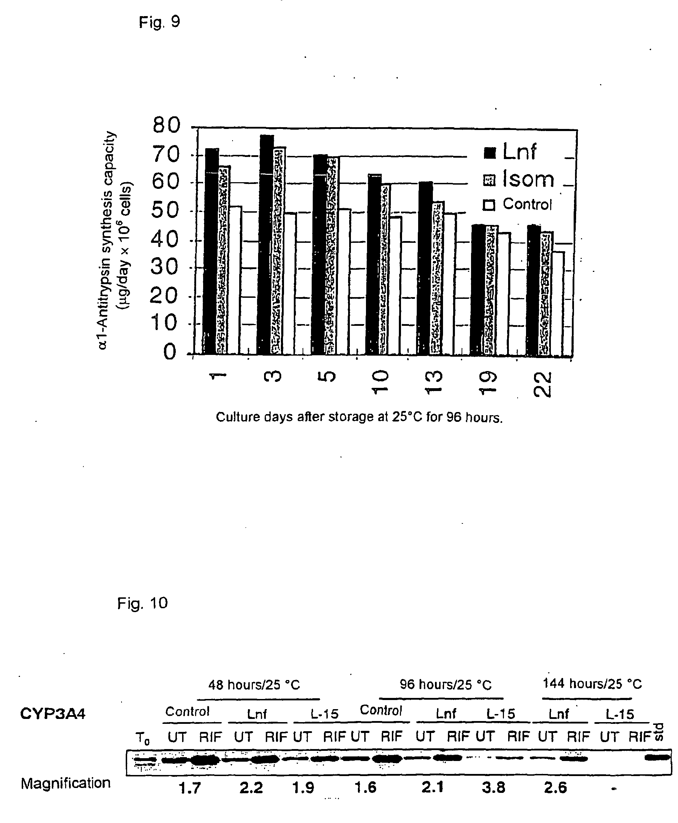

Human hepatocytes were maintained in Lnf or Isom at 25°C for 4 days. Subsequently, the cells were cultured at 37°C in Lnf, and the albumin synthesis capacity and α1-antitrypsin synthesis capacity were measured. The results are shown in Figs. 8 and 9.

As is apparent from Figs. 8 and 9, as compared with the control groups in which the cells were maintained and cultured at 37°C from the beginning, the cells that were cultured after having been maintained at 25°C for 4 days exhibited strong albumin synthesis capacity and strong α1-antitrypsin synthesis capacity.

### (5) CYP3A4 inducing capacity (human hepatocytes)

Human hepatocytes were maintained in Lnf or L-15 + 5% PEG at 25°C for 2, 4 or 6 days. Subsequently, the cells were cultured at 37°C in Lnf, and the CYP3A4 inducing capacity of the cells was measured. The results are shown in Fig. 10.

As is apparent from Fig. 10, as compared with the control groups in which the cells were maintained and cultured at 37°C from the beginning, the cells that were cultured after having been maintained in Lnf or L-15 + 5% PEG at 25°C for 2, 4, or 6 days exhibited strong CYP3A4 inducing capacity.

### Industrial Applicability

The method of the present invention enables long-term culturing of hepatocytes without permitting reduction in enzymatic activity or enzyme-inducing activity. Moreover, since the cells can be maintained at around room temperature for 1 to 6 days, the method of the invention is very useful for a long travel of hepatocytes after isolation.

## Claims

1. A method of culturing hepatocytes for a long term, **characterized in that** fresh hepatocytes are maintained in a medium at 15 to 30°C for 1 to 6 days, followed by culturing under physiological conditions.

2. The method of culturing hepatocytes for a long term according to claim 1, wherein the medium for fresh hepatocytes contains at least Lanford's medium.

3. The method of culturing hepatocytes for a long term according to claim 1 or 2, wherein the culturing under physiological conditions is performed at 37±1°C in a medium containing at least Lanford's medium.
